(19) Europäisches Patentamt
European Patent Office
Office européen des brevets

(11) Veröffentlichungsnummer: **0 185 345**
**A1**

(12) # EUROPÄISCHE PATENTANMELDUNG

(21) Anmeldenummer: **85116025.9**

(22) Anmeldetag: **16.12.85**

(51) Int. Cl.⁴: **C 07 D 487/04**
C 07 D 471/04, A 61 K 31/395
//(C07D487/04, 237:00, 235:00),
(C07D487/04, 239:00, 235:00),
(C07D487/04, 241:00, 235:00),
(C07D487/04, 251:00, 235:00),
(C07D487/04, 253:00, 235:00),
(C07D471/04, 235:00, 221:00)

(30) Priorität: **21.12.84 DE 3446778**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Heider, Joachim, Dr. Dipl.-Chem.**
**Am Hang 3**
**D-7951 Warthausen(DE)**

(72) Erfinder: **Hauel, Norbert, Dr. Dipl.-Chem.**
**Händelstrasse 12**
**D-7950 Biberach 1(DE)**

(72) Erfinder: **Austel, Volkhard, Dr. Dipl.-Chem.**
**Kapellenweg 7**
**D-7950 Biberach 1(DE)**

(72) Erfinder: **Noll, Klaus, Dr. Dipl.-Chem.**
**Im Schönblick 3**
**D-7951 Warthausen(DE)**

(72) Erfinder: **Bomhard, Andreas, Dr. Dipl.-Chem.**
**Dinglingerstrasse 9**
**D-7950 Biberach 1(DE)**

(72) Erfinder: **van Meel, Jacques, Dr.**
**Amriswilstrasse 7**
**D-7950 Biberach 1(DE)**

(72) Erfinder: **Diederen, Willi, Dr.**
**Haldenstrasse 1a**
**D-7950 Biberach 1(DE)**

(54) **Neue Imidazoderivate, ihre Herstellung und diese Verbindungen enthaltende Arzneimittel.**

(57) Die Erfindung betrifft neue Imidazoderivate der allgemeinen Formel

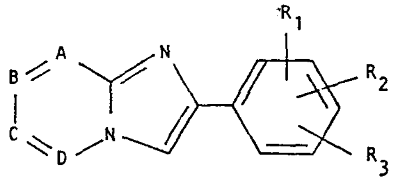

,(I)

in der
keiner, ein oder zwei der Reste A, B, C oder D ein Stickstoffatom,
ein weiterer der Reste A, B, C oder D eine gegebenenfalls durch ein Halogenatom, eine Alkylmercapto-, Alkylsulfonyloxy-, Amino-, Alkylamino-, Dialkylamino- oder Alkanoylaminogruppe substituierte Methingruppe,
ein weiterer der Reste der A, B, C oder D eine gegebenenfalls durch eine Alkylmercaptogruppe substituierte Methingruppe und
die übrigen der Reste A, B, C oder D Methingruppen,
$R_1$ und $R_2$ zusammen mit zwei dazwischen liegenden Kohlenstoffatomen des Phenylringes einen gegenbenenfalls durch eine Alkoxygruppe substituierten Phenylring und
$R_3$ ein Wasserstoffatom, eine Hydroxy-, Alkoxy- oder Alkylsulfonyloxygruppe oder
der erste der Reste $R_1$, $R_2$ oder $R_3$ eine Phenylalkoxy-, Alkylsulfonyloxy-, Alkylsulfonamido-, N-Alkyl-alkylsulfonamido-, Aminosulfonyl-, Alkylaminosulfonyl- oder Dialkylaminosulfonylgruppe oder in 4-Stellung eine Alkanoylamino-, Aminocarbonyl-, Alkylaminocarbonyl- oder Dialkylaminocarbonylgruppe oder

./...

im Falle der Imidazo[1,2-a]pyrimidine, Imidazo[1,2-b]pyridazine oder Imidazo[2,1-f][1,2,4]triazine oder, wenn einer oder zwei Reste A, B, C oder D einen der eingangs erwähnten substituierten Methinreste darstellen, in 4-Stellung auch eine Amino-, Hydroxy-, Methoxy- oder Cyanogruppe,
der zweite der Reste $R_1$, $R_2$ oder $R_3$ ein Wasserstoffatom, eine Hydroxy- oder Alkoxygruppe und der letzte der Reste $R_1$, $R_2$ oder $R_3$ ein Wasserstoffatom oder eine Alkoxygruppe, wobei der Alkylteil bei allen vorstehend erwähnten Resten 1 oder 2 Kohlenstoffatome enthalten kann, bedeuten, und deren Säureadditionssalze, insbesondere deren physiologisch verträgliche Säureadditionssalze mit anorganischen oder organischen Säuren.

Die neuen Verbindungen weisen wertvolle pharmakologische Eigenschaften auf, insbesondere antithrombotische und cardiovasculäre Eigenschaften wie eine cardiotonische und/oder eine Wirkung auf den Blutdruck, und lassen sich nach an und für sich bekannten Verfahren herstellen.

0185345

DR. KARL THOMAE GMBH
D-7950 Biberach 1

Case 5/916
Dr.Fl./Kp.

1.

Neue Imidazoderivate, ihre Herstellung und diese Verbindungen enthaltende Arzneimittel

---

Gegenstand der vorliegenden Erfindung sind neue Imidazoderivate der allgemeinen Formel

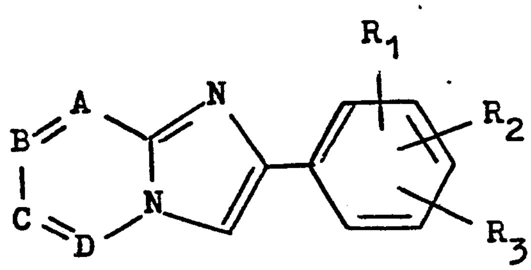

,(I)

und deren Säureadditionssalze, insbesondere deren physiologisch verträgliche Säureadditionssalze mit anorganischen oder organischen Säuren, welche wertvolle pharmakologische Eigenschaften aufweisen, insbesondere antithrombotische und cardiovasculäre Eigenschaften wie eine cardiotonische und/-oder eine Wirkung auf den Blutdruck.

In der obigen allgemeinen Formel I bedeutet

keiner, ein oder zwei der Reste A, B, C oder D ein Stickstoffatom,

ein weiterer der Reste A, B, C oder D eine gegebenenfalls durch ein Halogenatom, eine Alkylmercapto-, Alkylsulfonyloxy-, Amino-, Alkylamino-, Dialkylamino- oder Alkanoylaminogruppe substituierte Methingruppe, ein weiterer der Reste A, B, C oder D eine gegebenenfalls durch eine Alkylmercaptogruppe substituierte Methingruppe und die übrigen der Reste A, B, C oder D Methingruppen,

$R_1$ und $R_2$ zusammen mit zwei dazwischen liegenden Kohlenstoffatomen des Phenylringes einen gegenbenenfalls durch eine Alkoxygruppe substituierten Phenylring und

$R_3$ ein Wasserstoffatom, eine Hydroxy-, Alkoxy- oder Alkylsulfonyloxygruppe oder

der erste der Reste $R_1$, $R_2$ oder $R_3$ eine Phenylalkoxy-, Alkylsulfonyloxy-, Alkylsulfonamido-, N-Alkyl-alkylsulfonamido-, Aminosulfonyl-, Alkylaminosulfonyl- oder Dialkylaminosulfonylgruppe oder in 4-Stellung eine Alkanoylamino-, Aminocarbonyl-, Alkylaminocarbonyl- oder Dialkylaminocarbonylgruppe oder im Falle der Imidazo[1,2-a]pyrimidine, Imidazo[1,2-b]pyridazine oder Imidazo[2,1-f][1,2,4]triazine oder, wenn einer oder zwei der Reste A, B, C oder D einen der eingangs erwähnten substituierten Methinreste darstellen, in 4-Stellung auch eine Amino-, Hydroxy-, Methoxy- oder Cyanogruppe,

der zweite der Reste $R_1$, $R_2$ oder $R_3$ ein Wasserstoffatom, eine Hydroxy- oder Alkoxygruppe und der letzte der Reste $R_1$, $R_2$ oder $R_3$ ein Wasserstoffatom oder eine Alkoxygruppe, wobei der Alkylteil bei allen vorstehend erwähnten Resten 1 oder 2 Kohlenstoffatome enthalten kann.

Gegenstand der vorliegenden Erfindung sind somit insbesondere die entsprechend substituierten Imidazo[1.2-a]pyrimidine, Imidazo[1.2-c]pyrimidine, Imidazo[1.2-a][1.3.5]triazine, Imidazo[2.1-f][1.2.4]triazine, Imidazo[1.2-a]pyrazine, Imidazo[1.2-d][1.2.4]triazine, Imidazo[1.2-b][1.2.4]triazine, Imidazo[1.2-b]pyridazine, Imidazo[1.2-a]pyridine und Imidazo[2.1-c][1.2.4]triazine der obigen allgemeinen Formel I und deren Säureadditionssalze, insbesondere deren physiologisch verträgliche Säureadditionssalze.

Für die bei der Definition der Reste $R_1$ bis $R_3$ eingangs erwähnten Bedeutungen kommt somit

für $R_1$ und $R_2$ zusammen mit dem Phenylring insbesondere die Bedeutung der Naphth-1-yl-, Naphth-2-yl-, 2-Methoxy-naphth-1-yl-, 3-Methoxy-naphth-1-yl-, 4-Methoxy-naphth-1-yl-, 5-Methoxy-naphth-1-yl-, 6-Methoxy-naphth-1-yl-, 7-Methoxy-naphth-1-yl-, 8-Methoxy-naphth-1-yl-, 1-Methoxy-naphth-2-yl-, 3-Methoxy-naphth-2-yl-, 4-Methoxy-naphth-2-yl-, 5-Methoxy-naphth-2-yl-, 6-Methoxy-naphth-2-yl-, 7-Methoxy-naphth-2-yl-, 8-Methoxy-naphth-2-yl-, 2-Hydroxy-naphth-1-yl-, 3-Hydroxy-naphth-1-yl-, 4-Hydroxy-naphth-1-yl-, 6-Hydroxy-naphth-1-yl-, 2-Methansulfonyloxy-naphth-1-yl- oder 4-Methansulfonyloxy-naphth-1-yl-gruppe und

für $R_3$ die des Wasserstoffatoms, der Hydroxy-, Methoxy-, Ethoxy-, Methansulfonyloxy- oder Ethansulfonylgruppe oder

für $R_1$ die der Hydroxy-, Methoxy-, Ethoxy-, Benzyloxy-, 1-Phenylethoxy-, 2-Phenylethoxy-, Amino-, Acetamido-, Methylsulfonyloxy-, Ethylsulfonyloxy-, Aminosulfonyl-, Methylaminosulfonyl-, Ethylaminosulfonyl-, Dimethylaminosulfonyl-, Diethylaminosulfonyl-, N-Methyl-ethylaminosulfonyl-, Methylsulfonamido-, Ethylsulfonamido-, N-Methylmethylsulfonamido-, N-Ethyl-methylsulfonamido-, N-Methylethylsulfonamido-, N-Ethyl-ethylsulfonamido-, Cyano-, Aminocarbonyl-, Methylaminocarbonyl-, Ethylaminocarbonyl-, Dimethylaminocarbonyl-, Diethylaminocarbonyl- oder N-Methylethylaminocarbonylgruppe,

für $R_2$ die des Wasserstoffatoms, der Hydroxy-, Methoxy- oder Ethoxygruppe und

für $R_3$ die des Wasserstoffatoms, der Methoxy- oder Ethoxygruppe in Betracht.

Bevorzugte Verbindungen der obigen allgemeinen Formel I sind diejenigen, in denen

0, 1 oder 2 der Reste A, B, C oder D ein Stickstoffatom,

ein weiterer der Reste A, B, C oder D eine gegebenenfalls durch ein Chlor- oder Bromatom, eine Methylmercapto-, Methylsulfonyloxy-, Amino-, Methylamino-, Dimethylamino- oder Acetamidogruppe substituierte Methingruppe,

ein weiterer der Reste A, B, C oder D eine gegebenenfalls durch eine Methylmercaptogruppe substituierte Methingruppe und

die übrigen der Reste A, B, C oder D Methingruppen,

$R_1$ und $R_2$ zusammen mit zwei dazwischen liegenden Kohlenstoffatomen des Phenylringes einen gegebenfalls durch eine Methoxygruppe substituierten Phenylring und

$R_3$ ein Wasserstoffatom, eine Hydroxy-, Methoxy- oder Methansulfonyloxygruppe oder

der erste der Reste $R_1$, $R_2$ oder $R_3$ eine Acetamido-, Methylsulfonyloxy-, Methylsulfonamido-, N-Methyl-methylsulfonamido-, Aminosulfonyl-, Methylaminosulfonyl-, Dimethylaminosulfonyl-, Aminocarbonyl-, Methylaminocarbonyl- oder Dimethylaminocarbonylgruppe,

der zweite der Reste $R_1$, $R_2$ oder $R_3$ ein Wasserstoffatom, eine Hydroxy- oder Methoxygruppe und

der letzte der Reste $R_1$, $R_2$ oder $R_3$ ein Wasserstoffatom oder eine Methoxygruppe bedeuten, insbesondere jedoch diejenigen Verbindungen, in denen der erste der Reste in 2- oder 4-Position und der zweite der Reste in der verbleibenden 2- oder 4-Position steht, und deren Säureadditionssalze, insbesondere deren physiologisch verträgliche Säureadditionssalze.

Besonders bevorzugte Verbindungen der obigen allgemeinen Formel I sind jedoch diejenigen, in denen

0, 1 oder 2 der Reste A, B, C oder D Stickstoffatome und die übrigen der Reste A, B, C oder D Methingruppen,

$R_1$ in 2- oder 4-Stellung eine Methylsulfonyloxy-, Methylsulfonamido-, N-Methyl-methylsulfonamido-, Aminosulfonyl-, Methylaminosulfonyl- oder Dimethylaminosulfonylgruppe,

$R_2$ in der verbleibenden 2- oder 4-Stellung eine Methoxygruppe und

$R_3$ ein Wasserstoffatom darstellen, und deren Säureadditionssalze, insbesondere deren physiologisch verträgliche Säureadditionssalze.

Erfindungsgemäß erhält man die neuen Verbindungen nach folgenden Verfahren:

a) Umsetzung einer Verbindung der allgemeinen Formel

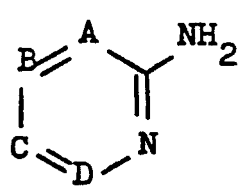

,(II)

in der
A, B, C und D wie eingangs definiert sind, mit einer Verbindung der allgemeinen Formel

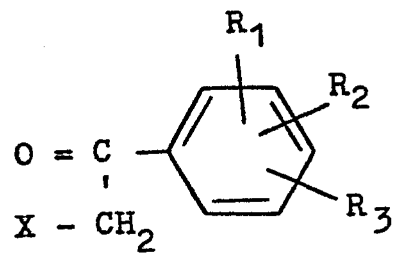

,(III)

in der
$R_1$, $R_2$ und $R_3$ wie eingangs definiert sind und
X eine nukleophile Austrittsgruppe wie ein Halogenatom, z.B. ein Chlor- oder Bromatom, darstellt.

Die Umsetzung wird zweckmäßigerweise in einem Lösungsmittel oder Lösungsmittelgemisch wie Ethanol, Isopropanol, Benzol, Glycol, Glycolmonomethylether, Dimethylformamid oder Dioxan beispielsweise bei Temperaturen zwischen 0 und 150°C, vorzugsweise bei Temperaturen zwischen 20 und 100°C, durchgeführt. Die Umsetzung kann jedoch auch ohne Lösungsmittel durchgeführt werden.

b) Zur Herstellung von Verbindungen der allgemeinen Formel I, in der einer der Reste $R_1$, $R_2$ oder $R_3$ eine Alkylsulfonyloxy-, Alkylsulfonamido- oder N-Alkyl-alkylsulfonamidogruppe darstellt:

Umsetzung einer Verbindung der allgemeinen Formel

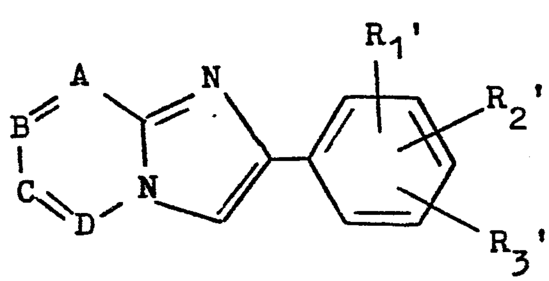

,(IV)

in der
A, B, C und D wie eingangs definiert sind und die Reste $R_1'$, $R_2'$ und $R_3'$ mit Ausnahme der Alkylsulfonyloxy-, Alkylsulfonamido- und N-Alkyl-alkylsulfonamidogruppe die für $R_1$, $R_2$ oder $R_3$ eingangs erwähnten Bedeutungen besitzen, wobei jedoch einer der Reste $R_1'$, $R_2'$ oder $R_3'$ eine Hydroxy-, Amino-, Methylamino- oder Ethylaminogruppe darstellen muß, mit einer Verbindung der allgemeinen Formel

$$R_4 - SO_2 - Y \qquad ,(V)$$

in der
$R_4$ eine Methyl- oder Ethylgruppe und
Y eine nukleophile Austrittsgruppe wie ein Halogenatom oder eine Alkoxygruppe, z.B. ein Chlor- oder Bromatom, eine Methoxy-, Ethoxy- oder Benzyloxygruppe, bedeuten.

Die Umsetzung wird zweckmäßigerweise in einem Lösungsmittel oder Lösungsmittelgemisch wie Wasser, Methanol, Ethanol, Isopropanol, Methylenchlorid, Ether, Tetrahydrofuran, Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

c) Zur Herstellung von Verbindungen der allgemeinen Formel I, in der mindestens einer der Reste $R_1$, $R_2$ oder $R_3$ eine Alkoxygruppe oder einer der Reste $R_1$, $R_2$ oder $R_3$ eine N-Alkyl-alkylsulfonamidogruppe darstellen:

Alkylierung einer Verbindung der allgemeinen Formel

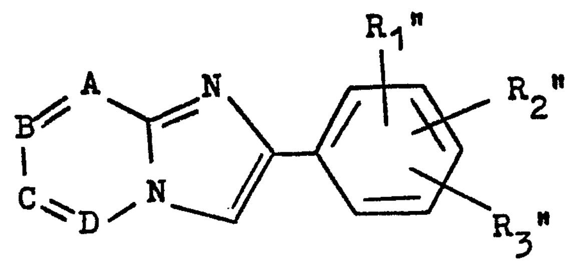

,(VI)

in der
A, B, C und D wie eingangs definiert sind und
$R_1$", $R_2$" und $R_3$" die für $R_1$, $R_2$ oder $R_3$ eingangs erwähnten Bedeutungen besitzen, wobei jedoch mindestens einer der Reste $R_1$", $R_2$" oder $R_3$" eine Hydroxy- und/oder eine Alkylsulfonamidogruppe darstellen muß, mit einer Verbindung der allgemeinen Formel

$$R_4 - Z \quad ,(VII)$$

in der
$R_4$ eine Methyl- oder Ethylgruppe und

Z eine nukleophile Austrittsgruppe wie ein Halogenatom oder eine Sulfonyloxygruppe, z.B. ein Chlor-, Brom- oder Jodatom, eine Methylsulfonyloxy-, Methoxysulfonyloxy- oder p-Toluolsulfonyloxygruppe, darstellen.

Die Umsetzung wird mit einem Alkylierungsmittel wie Methyljodid, Ethyljodid, Dimethylsulfat oder p-Toluolsulfonsäuremethylester zweckmäßigerweise in einem Lösungmittel wie Tetrahydrofuran, Dioxan, Dimethylformamid, Sulfolan, Dimethylsulfoxid oder Ethylenglycoldimethylether gegebenenfalls in Gegenwart eines säurebindenden Mittels wie Kaliumkarbonat, Kalium-tert.butylat, Triethylamin oder Pyridin, wobei die beiden letzteren gleichzeitig auch als Lösungsmittel verwendet werden können, bei Temperaturen zwischen 0 und 100°C, vorzugsweise bei Temperaturen zwischen 20 und 50°C, durchgeführt.

d) Zur Herstellung von Verbindungen der allgemeinen Formel I, in der einer der Reste $R_1$, $R_2$ oder $R_3$ eine Aminosulfonyl-, Alkylaminosulfonyl- oder Dialkylaminosulfonylgruppe darstellt:

Umsetzung einer Verbindung der allgemeinen Formel

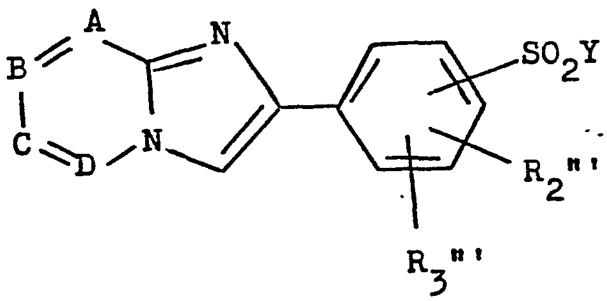

,(VIII)

in der
A, B, C und D wie eingangs definiert sind,
$R_2'''$ und $R_3'''$ die für $R_1$, $R_2$ und $R_3$ mit Ausnahme der Aminosulfonyl-, Alkylaminosulfonyl- oder Dialkylaminosulfonylgruppe eingangs erwähnten Bedeutungen besitzen und
Y eine nukleophile Austrittsgruppe wie ein Halogenatom oder eine Alkoxygruppe, z.B. ein Chloratom, eine Methoxy- oder Ethoxygruppe, darstellt, mit einem Amin der allgemeinen Formel

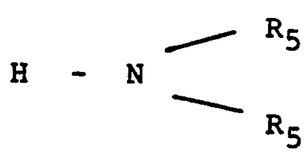

,(IX)

in der

$R_5$ jeweils ein Wasserstoffatom, eine Methyl- oder Ethylgruppe darstellen.

Die Umsetzung wird zweckmäßigerweise in einem Lösungsmittel oder Lösungsmittelgemisch wie Wasser, Methanol, Ethanol, Isopropanol, Methylenchlorid, Ether, Tetrahydrofuran, Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

e) Zur Herstellung von Verbindungen der allgemeinen Formel I, in der mindestens einer der Reste $R_1$ bis $R_3$ eine Amino-, Hydroxy- oder Aminocarbonylgruppe darstellt oder enthält:

Hydrolyse einer Verbindung der allgemeinen Formel

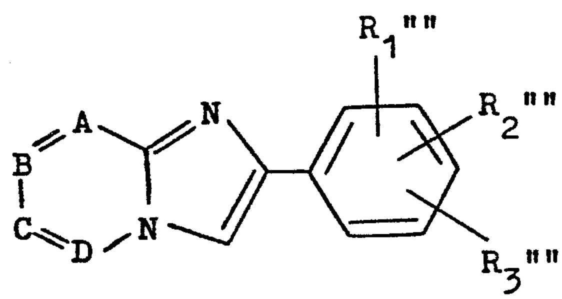

,(X)

in der

A, B, C und D mit Ausnahme der durch ein Halogenatom, eine Alkylmercapto-, Alkylsulfonyloxy- oder Phenylalkoxygruppe substituierte Methingruppe die für A, B, C und D eingangs erwähnten Bedeutungen aufweisen,

$R_1$"" bis $R_3$"" die für $R_1$ bis $R_3$ eingangs erwähnten Bedeutungen aufweisen, wobei jedoch einer der Reste $R_1$"" bis $R_3$"" eine Cyano-, Alkoxy-, Alkanoylamino- oder Alkylsulfonyloxygruppe enthalten oder darstellen muß.

Die Umsetzung wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid oder dem Alkalisalz eines entsprechenden Alkohols in der Schmelze oder in einem geeigneten Lösungmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Die Ätherspaltung wird jedoch vorzugsweise mit Bortribromid in einem Lösungsmittel wie Methylenchlorid bei Temperaturen zwischen -40°C und der Raumtemperatur durchgeführt.

Die partielle Hydrolyse der Cyanogruppe wird jedoch vorzugsweise mit konzentrierter Schwefelsäure oder mit einer Alkalilauge in Gegenwart von Wasserstoffperoxid, z.B. mit Natronlauge/Wasserstoffperoxid, zweckmäßigerweise bei Raumtemperatur durchgeführt.

f) Zur Herstellung von Verbindungen der allgemeinen Formel I, in der mindestens 2 der Reste A, B, C oder D Methingruppen oder mindestens einer der Reste $R_1$ bis $R_3$ ein Wasserstoffatom darstellen muß:

Reduktive Abspaltung eines oder zweier Reste von einer Verbindung der allgemeinen Formel

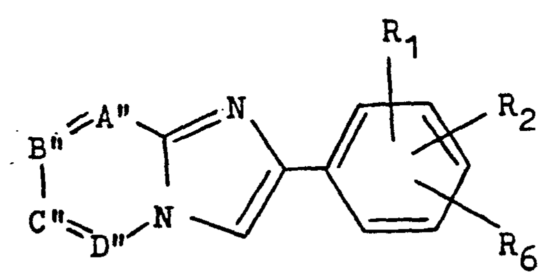

,(XI)

in der
$R_1$ und $R_2$ wie eingangs definiert sind,
$R_6$ die für $R_3$ eingangs erwähnten Bedeutungen besitzt und
A", B", C" oder D" die für A, B, C und D eingangs erwähnten Bedeutungen aufweisen, wobei jedoch mindestens einer der Reste $R_6$, A", B", C" oder D" einen reduktiv abspaltbaren Rest, z.B. ein Chlor-, Brom- oder Jodatom oder eine Alkylmercaptogruppe, darstellen oder enthalten muß.

Die reduktive Abspaltung erfolgt vorzugsweise mittels Hydrogenolyse. Diese wird zweckmäßigerweise in einem Lösungsmittel wie Methanol, Ethanol, Isopropanol, Eisessig, Essigester, Dimethylformamid oder Wasser gegebenenfalls in Gegenwart einer Mineralsäure wie Salzsäure oder Bromwasserstoffsäure bei Temperaturen zwischen -10°C und 100°C, vorzugsweise bei 0°C bis 60°C in Gegenwart eines Katalysators wie Raney-Nickel, Platin, Platinoxid- oder Palladium auf Kohle durchgeführt. - Eine gegebenenfalls vorhandene Benzyloxygruppe wird hierbei während der Umsetzung in eine Hydroxygruppe übergeführt.

Erhält man erfindungsgemäß eine Verbindung der allgemeinen Formel I, in der einer der Reste A, B, C oder D eine durch ein Halogenatom substituierte Methingruppe darstellt, so kann diese mittels Aminolyse in die entsprechende Aminoverbindung der allgemeinen Formel I übergeführt werden.

Die nachträgliche Aminolyse wird zweckmäßigerweise in einem Lösungsmittel oder Lösungsmittelgemisch wie Wasser, Methanol, Ethanol, Isopropanol, Methylenchlorid, Ether, Tetrahydrofuran, Dioxan, Dimethylformamid oder Benzol gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Die erfindungsgemäß erhaltenen Verbindungen der allgemeinen Formel I können gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II bis XI sind teilweise literaturbekannt bzw. erhält man nach literaturbekannten Verfahren.

So erhält man beispielsweise die als Ausgangsstoffe verwendeten Verbindungen der allgemeinen Formeln IV, VI, VIII, X und XI durch Umsetzung einer entsprechenden Aminoverbindung mit einem entsprechenden α-Brom-actophenon und gegebenenfalls anschließende Chlorsulfonierung.

Wie bereits eingangs erwähnt weisen die neuen Verbindungen der allgemeinen Formel I, deren 1H Tautomere und deren physiologisch verträgliche Säureadditionssalze überlegene pharmakologische Eigenschaften auf, insbesondere antithrombotische und cardiovasculäre Eigenschaften wie eine cardiotonische und/oder eine Wirkung auf den Blutdruck.

Beispielsweise wurden die Verbindungen

A = 2-(4-Methoxy-2-methylsulfonamido-phenyl)-imidazo-[1,2-a]pyrimidin,

B = 2-(2-Methoxy-4-methylsulfonyloxy-phenyl)-imidazo-[1,2-a]pyrimidin,

C = 2-(2-Methoxy-4-methylsulfonyloxy-phenyl)-imidazo-[1,2-a]pyrazin,

D = 2-(2-Methoxy-4-methylsulfonyloxy-phenyl)-imidazo-[1,2-b]pyridazin,

E = 2-(2-Methoxy-4-methylsulfonamido-phenyl)-imidazo[1,2-a]-pyridin und

F = 2-(2-Methoxy-4-aminosulfonyl-phenyl)-imidazo[1,2-a]pyrimidin

auf ihre biologischen Eigenschaften wie folgt untersucht:

Bestimmung der positiv inotropen Wirkung an despinalisierten Meerschweinchen

Die Untersuchungen wurden an despinalisierten Meerschweinchen durchgeführt. Die Tiere wurden mit 50 % $O_2$ + 50 % $N_2$ beatmet. Der arterielle Blutdruck wurde in der rechten Arteria Carotis mit einem Bell and Howell Druckwandler (4-327-I) gemessen. Für die Erfassung der positiv inotropen Wirkung wurde mit einem Kathetertipmanometer (Millar PR-249) der Druck in dem linken Ventrikel (LV) gemessen. Mittels eines Differenzierers wurde LV-dp/dtmax gewonnen. Die zu untersuchenden Substanzen wurden in eine Vena jugularis injiziert. Als Lösungsmittel diente 0,9 % NaCl+Polydiol 200 (1:1). Jede Substanz wurde an 3 Meerschweinchen überprüft. Die Dosen waren 0,1 - 3 mg/kg i.v.. Die nachfolgende Tabelle enthält die Mittelwerte bei 1 mg/kg i.v..

| Substanz | Dosis mg/kg i.v. | Zunahme in LV-dp/dtmax |
|---|---|---|
| A | 1 | 77 % |
| B | 1 | 74 % |
| C | 1 | 100 % |
| D | 1 | 56 % |
| E | 1 | 76 % |
| F | 1 | 56 % |

Die neuen Verbindungen sind gut verträglich, so konnte bei der Untersuchung der Substanzen A bis F bis zu einer Dosis von 3 mg/kg i.v. keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern und zum Teil durch die Blutdrucksenkung die Entleerung des Herzens erleichtern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 15 mg/kg Körpergewicht, vorzugsweise jedoch 3 - 10 mg/kg Körpergewicht.

Die nachfolgenden Beispiele sollen die Erfindung näher erläutern:

Beispiel 1

6-Chlor-2-(4-methoxy-2-methylsulfonamido-phenyl)-imidazo-[1,2-b]pyridazin

3,2 g (0,01 Mol) α-Brom-4-methoxy-2-methylsulfonamido-acetophenon und 2,6 g (0,02 Mol) 3-Amino-6-chlor-pyridazin werden in 30 ml Dimethylformamid gelöst, 16 Stunden lang bei Raumtemperatur gerührt und dann auf 100 ml Wasser gegossen. Das ausgefallene Produkt wird abgesaugt, mit Wasser gewaschen und im Vakuum bei 90°C getrocknet. Das Rohprodukt wird durch Chromatographie an Kieselgel (0,032-0,063 mm) gereinigt, Elutionsmittel: Methylenchlorid.

Ausbeute: 62,3 % der Theorie,

Schmelzpunkt: 208-209°C

| | | | | | | | | | | |
|---|---|---|---|---|---|---|---|---|---|---|
| Ber.: | C | 47,66 | H | 3,11 | N | 15,88 | Cl | 10,05 | S | 9,25 |
| Gef.: | | 47,67 | | 3,37 | | 16,08 | | 10,27 | | 9,20 |

Beispiel 2

2-(2-Methoxy-4-methylsulfonyloxy-phenyl)-6-methylsulfonyloxy-imidazo[1,2-b]pyridazin

0,3 g (1,17 mMol) 2-(2-Methoxy-4-hydroxyphenyl)-5H-imidazo-[1,2-b]pyridazin-6-on wird in 10 ml 1n Natronlauge gelöst. Bei 20°C wird unter Rühren 1,3 g (1,17 mMol) Methansulfonsäurechlorid zugetropft. Durch gleichzeitige Zugabe von 2n Natronlauge wird der pH-Wert bei 10-11 gehalten. Anschließend wird 3,5 Stunden bei 20°C nachgerührt, mit Eiswasser verrührt, abgesaugt und getrocknet. Durch Erwärmen mit 20 ml äthanolischer Salzsäure und anschließendem Verreiben mit Aceton und Äther wird gereinigt.

Ausbeute: 49,5 % der Theorie,

Schmelzpunkt: 243-248°C (Zers.)

Massenspektrum: $M^+$: 413 m/e

Beispiel 3

2-[4-Hydroxy-2-(N-methyl-methylsulfonamido)-phenyl]-imidazo-[1,2-a]pyridin

1,1 g (3,6 mMol) 2-(4-Hydroxy-2-methylsulfonamido-phenyl)-imidazo[1,2-a]pyridin werden in 10 ml Dimethylsulfoxid gelöst, mit 0,6 g (4,3 mMol) Kaliumcarbonat und 1,1 ml (18 mMol) Methyljodid versetzt. Die Mischung wird eine Stunde bei Raumtemperatur gerührt. Man gießt auf ca. 100 ml Eiswasser und saugt den ausgefallenen Niederschlag ab. Dieser wird mit Wasser gewaschen.
Ausbeute: 95,6 % der Theorie,
Schmelzpunkt: 177-180°C

| | | | | | | | | |
|---|---|---|---|---|---|---|---|---|
| Ber.: | C | 56,76 | H | 4,76 | N | 13,24 | S | 10,10 |
| Gef.: | | 55,02 | | 4,69 | | 12,82 | | 10,09 |

Beispiel 4

2-(4-Hydroxy-2-methylsulfonamido-phenyl)-imidazo-[1,2-a]-pyrimidin

Hergestellt aus 2-Amino-pyrimidin und α-Brom-4-hydroxy-2-methylsulfonamido-acetophenon analog Beispiel 1.
Ausbeute: 17,6 % der Theorie,
Schmelzpunkt: 264-266°C

| | | | | | | | | |
|---|---|---|---|---|---|---|---|---|
| Ber.: | C | 51,29 | H | 3,97 | N | 18,40 | S | 10,53 |
| Gef.: | | 51,16 | | 4,24 | | 18,48 | | 10,49 |

Beispiel 5

2-(4-Hydroxy-2-methylsulfonamido-phenyl)-imidazo[1,2-a]-pyridin

Hergestellt aus 2-Amino-pyridin und α-Brom-4-hydroxy-2-methylsulfonamido-acetophenon analog Beispiel 1.
Ausbeute: 13,3 % der Theorie,
Schmelzpunkt: 250-253°C

| | | | | | | | | |
|---|---|---|---|---|---|---|---|---|
| Ber.: | C | 55,44 | H | 4,32 | N | 13,85 | S | 10,57 |
| Gef.: | | 55,16 | | 4,56 | | 13,67 | | 10,70 |

Beispiel 6

2-(4-Methoxy-2-methylsulfonamido-phenyl)-imidazo[1,2-a]-pyrimidin

Hergestellt aus 2-Amino-pyrimidin und α-Brom-2-methylsulfonamido-4-methoxy-acetophenon analog Beispiel 1.
Ausbeute: 46,9 % der Theorie,
Schmelzpunkt: 252-254°C

| | | | | | | | | |
|---|---|---|---|---|---|---|---|---|
| Ber.: | C | 52,81 | H | 4,43 | N | 17,59 | S | 10,07 |
| Gef.: | | 52,64 | | 4,21 | | 17,77 | | 10,02 |

Beispiel 7

2-(2-Hydroxy-4-methylsulfonyloxy-phenyl)-imidazo[1,2-a]-pyrimidin

Hergestellt aus 2-Amino-pyrimidin und α-Brom-2-hydroxy-4-methylsulfonyloxy-acetophenon analog Beispiel 1.

Ausbeute: 32,7 % der Theorie,
Schmelzpunkt: 244-246°C

| | | | | | | | | |
|---|---|---|---|---|---|---|---|---|
| Ber.: | C | 51,14 | H | 3,63 | N | 13,76 | S | 10,50 |
| Gef.: | | 51,08 | | 3,69 | | 13,52 | | 10,55 |

Beispiel 8

2-(2-Methoxy-4-methylsulfonyloxy-phenyl)-imidazo[1,2-a]-pyrimidin

Hergestellt aus 2-Amino-pyrimidin und α-Brom-2-methoxy-4-methylsulfonyloxy-acetophenon analog Beispiel 1.
Ausbeute: 6,2 % der Theorie,
Schmelzpunkt: 182-184°C

| | | | | | | | | |
|---|---|---|---|---|---|---|---|---|
| Ber.: | C | 52,66 | H | 4,10 | N | 13,16 | S | 10,04 |
| Gef.: | | 52,40 | | 3,99 | | 13,00 | | 10,11 |

Beispiel 9

2-(2-Methoxy-4-methylsulfonyloxy-phenyl)-imidazo[1,2-a]-pyridin

Hergestellt aus 2-Amino-pyridin und α-Brom-2-methoxy-4-methylsulfonyloxy-acetophenon analog Beispiel 1.
Ausbeute: 65,5 % der Theorie,
Schmelzpunkt: 143-146°C

| | | | | | | | | |
|---|---|---|---|---|---|---|---|---|
| Ber.: | C | 56,58 | H | 4,43 | N | 8,79 | S | 10,07 |
| Gef.: | | 56,84 | | 4,23 | | 8,76 | | 10,02 |

Beispiel 10

2-(4-Methoxy-2-methylsulfonamido-phenyl)-imidazo[1,2-a]-pyridin

Hergestellt aus 2-Amino-pyridin und α-Brom-4-methoxy-2-methylsulfonylamido-acetophenon analog Beispiel 1.
Ausbeute: 6,2 % der Theorie,
Schmelzpunkt: 248-251°C
Analyse des Hydrochlorids:

| | | | | | | | | | | |
|---|---|---|---|---|---|---|---|---|---|---|
| Ber.: | C | 50,85 | H | 4,55 | N | 11,86 | Cl | 10,01 | S | 9,05 |
| Gef.: | | 51,11 | | 4,34 | | 11,55 | Cl | 9,97 | | 9,01 |

Beispiel 11

2-(4-Dimethylaminosulfonyl-phenyl)-imidazo[1,2-a]pyrimidin

Hergestellt aus 2-Amino-pyrimidin und α-Brom-4-dimethyl-aminosulfonyl-acetophenon analog Beispiel 1.
Ausbeute: 45,6 % der Theorie,
Schmelzpunkt: 280-283°C

| | | | | | | | | |
|---|---|---|---|---|---|---|---|---|
| Ber.: | C | 55,61 | H | 4,67 | N | 18,53 | S | 10,61 |
| Gef.: | | 55,41 | | 4,81 | | 18,18 | | 10,88 |

Beispiel 12

2-(4-Cyano-phenyl)-imidazo[1,2-a]pyrimidin

Hergestellt aus 2-Amino-pyrimidin und α-Brom-4-cyano-acetophenon analog Beispiel 1.

Ausbeute: 56,8 % der Theorie,
Schmelzpunkt: 335-336°C

| | | | | | | |
|---|---|---|---|---|---|---|
| Ber.: | C | 70,90 | H | 3,66 | N | 25,44 |
| Gef.: | | 70,99 | | 3,70 | | 25,54 |

Beispiel 13

2-(4-Methylsulfonamido-phenyl)-imidazo[1,2-a]pyrimidin

Hergestellt aus 2-Amino-pyrimidin und α-Brom-4-methylsulfonamido-acetophenon analog Beispiel 1.
Ausbeute: 22,6 % der Theorie,
Schmelzpunkt: 260-266°C

| | | | | | | | | |
|---|---|---|---|---|---|---|---|---|
| Ber.: | C | 54,15 | H | 4,20 | N | 19,43 | S | 11,12 |
| Gef.: | | 53,82 | | 4,27 | | 19,66 | | 11,07 |

Beispiel 14

2-(4-Methylsulfonyloxy-phenyl)-imidazo[1,2-a]pyrimidin

Hergestellt aus 2-Amino-pyrimidin und α-Brom-4-methylsulfonyloxy-acetophenon analog Beispiel 1.
Ausbeute: 22,4 % der Theorie,
Schmelzpunkt: 238-240°C

| | | | | | | | | |
|---|---|---|---|---|---|---|---|---|
| Ber.: | C | 53,97 | H | 3,83 | N | 14,53 | S | 11,08 |
| Gef.: | | 53,77 | | 3,71 | | 14,65 | | 10,82 |

Beispiel 15

2-(4-Methoxy-phenyl)-imidazo[1,2-a]pyrimidin

Hergestellt aus 2-Amino-pyrimidin und α-Brom-4-methoxy-acetophenon analog Beispiel 1.
Ausbeute: 42,2 % der Theorie,
Schmelzpunkt: 198-200°C

| | | | | | | |
|---|---|---|---|---|---|---|
| Ber.: | C | 69,32 | H | 4,92 | N | 18,66 |
| Gef.: | | 69,10 | | 4,81 | | 18,80 |

Beispiel 16

7-(4-Methylsulfonyloxy-2-methoxy-phenyl)-imidazo[2,1-c]-[1,2,4]triazin

Hergestellt aus 3-Amino[1,2,4]triazin und α-Brom-4-methylsulfonyloxy-2-methoxy-acetophenon analog Beispiel 1.
Ausbeute: 64 % der Theorie,
Schmelzpunkt: 194-196°C

| | | | | | | |
|---|---|---|---|---|---|---|
| Ber.: | C | 48,74 | H | 3,78 | S | 10,01 |
| Gef.: | | 48,66 | | 3,67 | | 9,98 |

Beispiel 17

7-(4-Methylsulfonyloxy-2-methoxy-phenyl)-imidazo[1,2-a]-[1,3,5]triazin

Hergestellt aus 2-Amino[1,3,5]triazin und α-Brom-4-methylsulfonyloxy-2-methoxy-acetophenon analog Beispiel 1.
Ausbeute: 8,5 % der Theorie,

$R_f$-Wert des amorphen Produkts: 0,18 (Merck-Kieselgel, Laufmittel: $CH_2Cl_2/C_2H_5OH$ (10:1))
Massenspektrum: $M^+$ = 320 m/e

Beispiel 18

2-(4-Methylsulfonyloxy-2-methoxy-phenyl)-imidazo[1,2-a]-pyrazin

Hergestellt aus 2-Amino-pyrazin und α-Brom-4-methylsulfonyl-oxy-2-methoxy-acetophenon analog Beispiel 1.
Ausbeute: 3,4 % der Theorie,
Schmelzpunkt: 185-187°C

| | | | | | | |
|---|---|---|---|---|---|---|
| Ber.: | C | 52,60 | H | 4,10 | N | 13,16 |
| Gef.: | | 52,40 | | 3,87 | | 12,97 |

Beispiel 19

2-(4-Methoxy-2-methylsulfonamido-phenyl)-imidazo[1,2-b]-pyridazin

Hergestellt aus 3-Amino-pyridazin-hydrochlorid und α-Brom-4-methoxy-2-methylsulfonamido-acetophenon analog Beispiel 1.
Ausbeute: 10 % der Theorie,
Schmelzpunkt: 198-200°C

| | | | | | | | | |
|---|---|---|---|---|---|---|---|---|
| Ber.: | C | 52,81 | H | 4,43 | N | 17,60 | S | 10,07 |
| Gef.: | | 53,08 | | 4,28 | | 17,69 | | 10,73 |

Beispiel 20

2-(2-Methoxy-4-methylsulfonyloxy-phenyl)-imidazo[1,2-b]-pyridazin

Hergestellt aus 3-Amino-pyridazin-hydrochlorid und α-Brom-2-methoxy-4-methylsulfonyloxy-acetophenon analog Beispiel 1.
Ausbeute: 7 % der Theorie,
Schmelzpunkt: 150-151°C

| | | | | | | | | |
|---|---|---|---|---|---|---|---|---|
| Ber.: | C | 52,65 | H | 4,10 | N | 13,16 | S | 10,04 |
| Gef.: | | 52,88 | | 4,29 | | 13,28 | | 9,94 |

Beispiel 21

6-Chlor-2-(2-methoxy-4-methylsulfonyloxy-phenyl)-imidazo-[1,2-b]pyridazin

Hergestellt aus 3-Amino-6-chlor-pyridazin und α-Brom-2-methoxy-4-methylsulfonyloxy-acetophenon analog Beispiel 1.
Ausbeute: 31,1 % der Theorie,
Schmelzpunkt: 182-183°C

| | | | | | | | | | | |
|---|---|---|---|---|---|---|---|---|---|---|
| Ber.: | C | 47,53 | H | 3,42 | N | 11,88 | Cl | 10,02 | S | 9,06 |
| Gef.: | | 47,51 | | 3,30 | | 11,58 | | 9,86 | | 8,92 |

Beispiel 22

2-(4-Acetamido-phenyl)-6-chlor-imidazo[1,2-b]pyridazin

Hergestellt aus 3-Amino-6-chlor-pyridazin und α-Brom-4-acetamido-acetophenon analog Beispiel 1.

Ausbeute: 73,3 % der Theorie,
Schmelzpunkt: 308-312°C

| | | | | | | | | |
|---|---|---|---|---|---|---|---|---|
| Ber.: | C | 58,65 | H | 3,87 | N | 19,54 | Cl | 12,37 |
| Gef.: | | 58,92 | | 3,98 | | 19,70 | | 12,19 |

Beispiel 23

6-(4-Methoxy-phenyl)-2,4-bis(methylmercapto)-imidazo[2,1-f]-[1,2,4]triazin

Hergestellt aus 6-Amino-3,5-bis(methylmercapto)-[1,2,4]triazin und α-Brom-4-methoxy-acetophenon in Dimethylformamid analog Beispiel 1.
Ausbeute: 71,25 % der Theorie,
Schmelzpunkt: 167-169°C

| | | | | | | | | |
|---|---|---|---|---|---|---|---|---|
| Ber.: | C | 52,83 | H | 4,43 | N | 17,60 | S | 20,19 |
| Gef.: | | 52,58 | | 4,50 | | 17,43 | | 20,06 |

Beispiel 24

6-(4-Hydroxy-2-methoxy-phenyl)-2,4-bis(methylmercapto)-imidazo[2,1-f][1,2,4]triazin

Hergestellt aus 6-Amino-3,5-bis(methylmercapto)-[1,2,4]triazin und α-Chlor-2-methoxy-4-hydroxy-acetophenon analog Beispiel 1.
Ausbeute: 46,4 % der Theorie,
Schmelzpunkt: 189-191°C

| | | | | | | | | |
|---|---|---|---|---|---|---|---|---|
| Ber.: | C | 50,30 | H | 4,22 | N | 16,76 | S | 19,18 |
| Gef.: | | 50,10 | | 4,11 | | 16,78 | | 19,18 |

Beispiel 25

2-(4-Dimethylaminosulfonyl-phenyl)-imidazo[1,2-a]pyrimidin

2,9 g (0,01 Mol) 2-(4-Chlorsulfonyl-phenyl)-imidazo[1,2-a]-pyrimidin werden in 60 ml einer 40%igen Lösung von Dimethylamin in Wasser suspendiert. Man erhitzt 30 Minuten lang auf 50°C, verdünnt mit 50 ml Wasser, extrahiert mit Essigester, trocknet den Essigesterextrakt und dampft zur Trockne ein. Der Rückstand wird aus Dimethylformamid umkristallisiert.
Ausbeute: 68,5 % der Theorie,
Schmelzpunkt: 280-283°C

| | | | | | | | | |
|---|---|---|---|---|---|---|---|---|
| Ber.: | C | 55,61 | H | 4,67 | N | 18,53 | S | 10,61 |
| Gef.: | | 55,41 | | 4,81 | | 18,28 | | 10,88 |

Beispiel 26

2-(2-Methoxy-4-methylsulfonamido-phenyl)-imidazo[1,2-a]pyrimidin

Hergestellt aus 2-Amino-pyrimidin und α-Brom-2-methoxy-4-methylsulfonamido-acetophenon analog Beispiel 1.
Ausbeute: 58 % der Theorie,
Schmelzpunkt: 264-266°C

| | | | | | | | | |
|---|---|---|---|---|---|---|---|---|
| Ber.: | C | 52,81 | H | 4,43 | N | 17,59 | S | 10,07 |
| Gef.: | | 52,60 | | 4,47 | | 17,39 | | 10,10 |

Beispiel 27

2-(2-Methoxy-4-methylsulfonamido-phenyl)-imidazo[1,2-a]pyridin

Hergestellt aus 2-Amino-pyridin und α-Brom-2-methoxy-4-methylsulfonamido-acetophenon analog Beispiel 1.
Ausbeute: 84,8 % der Theorie,
Schmelzpunkt: 235-237°C

| | | | | | | | | |
|---|---|---|---|---|---|---|---|---|
| Ber.: | C | 56,76 | H | 4,76 | N | 13,24 | S | 10,10 |
| Gef.: | | 56,56 | | 4,90 | | 13,17 | | 10,02 |

Beispiel 28

6-Chlor-2-(4-acetamido-2-methoxy-phenyl)-imidazo[1,2-b]-pyridazin

Hergestellt aus 3-Amino-6-chlor-pyridazin und α-Brom-4-acetamido-2-methoxy-acetophenon analog Beispiel 1.
Ausbeute: 73 % der Theorie,
Schmelzpunkt: 267-269°C (Zersetzung)

| | | | | | | | | |
|---|---|---|---|---|---|---|---|---|
| Ber.: | C | 56,88 | H | 4,14 | N | 17,69 | Cl | 11,19 |
| Gef.: | | 56,90 | | 4,38 | | 17,62 | | 11,25 |

Beispiel 29

2-(4-Methylsulfonamido-phenyl)-imidazo[1,2-b]pyridazin

Hergestellt aus 2-(4-Amino-phenyl)-imidazo[1,2-b]pyridazin und Methansulfonsäurechlorid in Pyridin analog Beispiel 2.
Ausbeute: 94 % der Theorie,
Schmelzpunkt: 240-245°C (Zersetzung)

| | | | | | | | | |
|---|---|---|---|---|---|---|---|---|
| Ber.: | C | 54,16 | H | 4,20 | N | 19,44 | S | 11,12 |
| Gef.: | | 53,70 | | 4,14 | | 19,64 | | 11,28 |

Beispiel 30

6-Chlor-(4-amino-phenyl)-imidazo[1,2-b]pyridazin

Hergestellt aus 6-Chlor-3-amino-pyridazin und α-Brom-4-amino-acetophenon analog Beispiel 1.
Ausbeute: 20 % der Theorie,
Schmelzpunkt: 220-228°C
NMR-Spektrum (Dimethylsulfoxid): aromatisches H: 6,6 und 7,7 ppm (2d, 4H)
H in 3-Stellung: 8,6 ppm (s, 1H)
H in 8- und 9-Stellung: 7,2 und 8,1 ppm (2d, 2H)
UV-Spektrum (Ethanol): 223 nm (0,47), 265 nm (0,31)
385 nm (0,25)

Beispiel 31

2-(4-Acetamido-phenyl)-imidazo[1,2-b]pyridazin

Hergestellt aus 3-Amino-pyridazin und α-Brom-4-acetamido-acetophenon analog Beispiel 1.
Ausbeute: 72 % der Theorie,
Schmelzpunkt: 300-305°C (Zersetzung)
$C_{14}H_{12}N_4O \times 0,75\ H_2O$

| | | | | | | |
|---|---|---|---|---|---|---|
| Ber.: | C | 63,26 | H | 5,12 | N | 21,08 |
| Gef.: | | 63,37 | | 4,87 | | 21,26 |

Beispiel 32

2-(4-Amino-phenyl)-imidazo[1,2-b]pyridazin

Hergestellt aus 3-Amino-pyridazin und α-Brom-4-amino-

acetophenon analog Beispiel 1.
Ausbeute: 69 % der Theorie,
Schmelzpunkt: 195-202°C

| | | | | | | |
|---|---|---|---|---|---|---|
| Ber.: | C | 68,57 | H | 4,80 | N | 26,67 |
| Gef.: | | 68,57 | | 4,70 | | 26,52 |

Beispiel 33

2-(2,4-Dimethoxy-5-dimethylaminosulfonyl-phenyl)-imidazo-[1,2-a]pyrimidin

Hergestellt aus 2-(2,4-Dimethoxy-5-chlorsulfonyl-phenyl)-imidazo[1,2-a]pyrimidin (hergestellt aus 2-(2,4-Dimethoxyphenyl[1,2-a]pyrimidin und Chlorsulfonsäure) und 40%igem Dimethylamin analog Beispiel 25.
Ausbeute: 42 % der Theorie,
Schmelzpunkt: 246-248°C

| | | | | | | | | |
|---|---|---|---|---|---|---|---|---|
| Ber.: | C | 50,51 | H | 5,28 | N | 14,72 | S | 8,85 |
| Gef.: | | 50,30 | | 5,28 | | 14,74 | | 8,86 |

Beispiel 34

2-(2-Methoxy-4-aminocarbonyl-phenyl)-imidazo[1,2-a]pyrimidin

Hergestellt aus 2-Amino-pyrimidin und α-Brom-2-methoxy-4-aminocarbonyl-acetophenon analog Beispiel 1.
Ausbeute: 96,2 % der Theorie,
Schmelzpunkt: 290-291°C

| | | | | | | |
|---|---|---|---|---|---|---|
| Ber.: | C | 58,74 | H | 4,93 | N | 19,57 |
| Gef.: | | 58,60 | | 4,74 | | 19,36 |

Beispiel 35

2-(2-Methoxy-4-aminocarbonyl-phenyl)-imidazo[1,2-a]pyridin

Hergestellt aus 2-Amino-pyridin und α-Brom-2-methoxy-4-aminocarbonyl-acetophenon analog Beispiel 1.
Ausbeute: 56,6 % der Theorie,
Schmelzpunkt: 263-266°C

| | | | | | | |
|---|---|---|---|---|---|---|
| Ber.: | C | 67,41 | H | 4,90 | N | 15,72 |
| Gef.: | | 67,14 | | 4,97 | | 15,53 |

Beispiel 36

2-(2-Methoxy-4-aminosulfonyl-phenyl)-imidazo[1,2-a]pyrimidin

Hergestellt aus 2-Amino-pyrimidin und α-Brom-2-methoxy-4-aminosulfonyl-acetophenon analog Beispiel 1.
Ausbeute: 51,6 % der Theorie,
Schmelzpunkt: 294-296°C

| | | | | | | | | |
|---|---|---|---|---|---|---|---|---|
| Ber.: | C | 51,31 | H | 3,97 | N | 18,41 | S | 10,53 |
| Gef.: | | 50,99 | | 3,98 | | 18,36 | | 10,37 |

Beispiel 37

2-(2-Methoxy-4-methylaminosulfonyl-phenyl)-imidazo[1,2-a]-pyrimidin

Hergestellt aus 2-Amino-pyrimidin und α-Brom-2-methoxy-4-methylaminosulfonyl-acetophenon analog Beispiel 1.
Ausbeute: 41,2 % der Theorie,
Schmelzpunkt: 304-306°C

| | | | | | | | | |
|---|---|---|---|---|---|---|---|---|
| Ber.: | C | 52,81 | H | 4,43 | N | 17,59 | S | 10,07 |
| Gef.: | | 52,55 | | 4,36 | | 17,63 | | 9,91 |

Beispiel 38

2-(2-Methoxy-4-methylaminosulfonyl-phenyl)-imidaz[1,2-a]-pyridin

Hergestellt aus 2-Amino-pyridin und α-Brom-2-methoxy-4-methylaminosulfonyl-acetophenon analog Beispiel 1.
Ausbeute: 44,4 % der Theorie,
Schmelzpunkt: 241-244°C

| | | | | | | | | |
|---|---|---|---|---|---|---|---|---|
| Ber.: | C | 56,76 | H | 4,76 | N | 13,24 | S | 10,10 |
| Gef.: | | 56,86 | | 4,84 | | 13,09 | | 10,11 |

Beispiel 39

2-(2-Methoxy-4-cyano-phenyl)-imidazo[1,2-a]pyrimidin

Hergestellt aus 2-Amino-pyrimidin und α-Brom-2-methoxy-4-cyano-acetophenon analog Beispiel 1.
Ausbeute: 56 % der Theorie,
Schmelzpunkt: 289-291°C

| | | | | | | |
|---|---|---|---|---|---|---|
| Ber.: | C | 67,19 | H | 4,03 | N | 22,39 |
| Gef.: | | 67,00 | | 3,99 | | 22,46 |

Beispiel 40

2-(2-Methoxy-4-cyano-phenyl)-imidazo[1,2-a]pyridin

Hergestellt aus 2-Amino-pyridin und α-Brom-2-methoxy-4-cyano-acetophenon analog Beispiel 1.
Ausbeute: 44 % der Theorie,
Schmelzpunkt: 210-213°C

| | | | | | | |
|---|---|---|---|---|---|---|
| Ber.: | C | 72,28 | H | 4,45 | N | 16,86 |
| Gef.: | | 72,50 | | 4,59 | | 17,12 |

Beispiel 41

2-(3-Dimethylaminosulfonyl-4-methoxy-phenyl)-imidazo[1,2-a]-pyrimidin-hydrobromid

---

Hergestellt aus 2-(4-Methoxy-3-chlorsulfonyl-phenyl)-imidazo-[1,2-a]pyrimidin (hergestellt aus 2-(4-Methoxy-phenyl)-imidazo[1,2-a]pyrimidin und Chlorsulfonsäure) und 40%igem Methylamin analog Beispiel 25.
Ausbeute: 59,8 % der Theorie,
Schmelzpunkt: 205-210°C

| | | | | | | | | | | |
|---|---|---|---|---|---|---|---|---|---|---|
| Ber.: | C | 43,59 | H | 4,14 | N | 13,55 | S | 7,76 | Br | 19,33 |
| Gef.: | | 43,86 | | 3,96 | | 13,60 | | 8,25 | | 19,31 |

Beispiel 42

2-(2-Methoxy-4-dimethylaminosulfonyl-phenyl)-imidazo[1,2-a]-pyrimidin

---

Hergestellt aus 2-Amino-pyrimidin und α-Brom-2-methoxy-4-dimethylaminosulfonyl-acetophenon analog Beispiel 1.
Ausbeute: 54,2 % der Theorie,
Schmelzpunkt: 233-236°C

| | | | | | | | | |
|---|---|---|---|---|---|---|---|---|
| Ber.: | C | 54,20 | H | 4,85 | N | 16,85 | S | 9,65 |
| Gef.: | | 54,50 | | 4,74 | | 16,94 | | 9,73 |

Beispiel 43

2-(2-Methoxy-4-dimethylaminosulfonyl-phenyl)-imidazo[1,2-a]-pyridin

---

Hergestellt aus 2-Amino-pyridin und α-Brom-2-methoxy-4-dimethylaminosulfonyl-acetophenon analog Beispiel 1.
Ausbeute: 65,1 % der Theorie,
Schmelzpunkt: 223-225°C

| | | | | | | | | |
|---|---|---|---|---|---|---|---|---|
| Ber.: | C | 57,99 | H | 5,17 | N | 12,68 | S | 9,67 |
| Gef.: | | 57,75 | | 5,02 | | 12,46 | | 9,67 |

Beispiel 44

2-(2-Methylsulfonylamino-4-methylsulfonyloxy-phenyl)-imidazo-[1,2-a]pyrimidin

Hergestellt aus α-Brom-2-methylsulfonylamino-4-methylsulfonyloxy-acetophenon und 2-Amino-pyrimidin analog Beispiel 1.
Ausbeute: 40,5 % der Theorie,
Schmelzpunkt: 276-277°C

| | | | | | | | | |
|---|---|---|---|---|---|---|---|---|
| Ber.: | C | 43,97 | H | 3,69 | N | 14,65 | S | 16,77 |
| Gef.: | | 43,79 | | 3,75 | | 14,70 | | 16,58 |

Beispiel 45

6-Chlor-2-(1-methoxy-naphth-2-yl)-imidazo[1,2-a]pyridin

Hergestellt aus 2-Amino-5-chlor-pyridin und α-Brom-2-(1-methoxy)-acetophenon analog Beispiel 1.
Ausbeute: 57 % der Theorie,
Schmelzpunkt: 155-156°C

| | | | | | | |
|---|---|---|---|---|---|---|
| Ber.: | C | 70,02 | H | 4,24 | N | 9,07 |
| Gef.: | | 69,97 | | 4,18 | | 9,01 |

Beispiel 46

7-(Naphth-2-yl)-imidazo[2,1-c][1,2,4]triazin

Hergestellt aus α-Brom-2-acetonaphthon und 3-Amino[1,2,4]-triazin analog Beispiel 1.
Ausbeute: 20,0 % der Theorie,
Schmelzpunkt: 215-216°C

| | | | | | | |
|---|---|---|---|---|---|---|
| Ber.: | C | 73,16 | H | 4,09 | N | 22,75 |
| Gef.: | | 72,98 | | 4,15 | | 22,48 |

Beispiel 47

2-(1-Methoxy-naphth-4-yl)-imidazo[1,2-a]pyrimidin

Hergestellt aus α-Brom-4-(1-methoxy)-acetonaphthon und 2-Amino-pyrimidin analog Beispiel 1.
Ausbeute: 11 % der Theorie,
Schmelzpunkt: 188-189°C

| | | | | | | |
|---|---|---|---|---|---|---|
| Ber.: | C | 74,17 | H | 4,76 | N | 15,26 |
| Gef.: | | 74,08 | | 4,76 | | 15,28 |

Beispiel 48

2-(1-Methylsulfonyloxy-naphth-4-yl)-imidazo[1,2-a]pyrimidin

Hergestellt aus 2-(1-Hydroxy-naphth-4-yl)-imidazo[1,2-a]pyrimidin und Methansulfonsäurechlorid analog Beispiel 2.
Ausbeute: 26 % der Theorie,
Schmelzpunkt: 199-200°C

| | | | | | | |
|---|---|---|---|---|---|---|
| Ber.: | C | 60,16 | H | 3,86 | N | 12,38 |
| Gef.: | | 60,01 | | 3,81 | | 12,41 |

Beispiel 49

2-(1-Methoxy-naphth-2-yl)-imidazo[1,2-a]pyridin

Hergestellt aus 6-Chlor-2-(1-methoxy-naphth-2-yl)-imidazo-[1,2-a]pyridin analog Beispiel 54.
Ausbeute: 6 % der Theorie,
Schmelzpunkt: 146-148°C

| | | | | | | |
|---|---|---|---|---|---|---|
| Ber.: | C | 78,81 | H | 5,14 | N | 10,21 |
| Gef.: | | 78,93 | | 5,23 | | 10,23 |

Beispiel 50

2-(2-Methoxy-naphth-6-yl)-imidazo[1,2-a]pyrimidin

Hergestellt aus α-Brom-6-(2-methoxy)-acetonaphthon und 2-Amino-pyrimidin analog Beispiel 1.
Ausbeute: 22 % der Theorie,
Schmelzpunkt: 268-269°C

| | | | | | | |
|---|---|---|---|---|---|---|
| Ber.: | C | 74,17 | H | 4,76 | N | 15,26 |
| Gef.: | | 74,07 | | 5,00 | | 15,22 |

Beispiel 51

2-(1-Hydroxy-naphth-4-yl)-imidazo[1,2-a]pyrimidin

1,38 g (5 mMol) 2-(1-Methoxy-naphth-4-yl)-imidazo[1,2-a]-pyrimidin werden in 50 ml Methylenchlorid suspendiert, auf -40°C gekühlt und unter Rühren tropfenweise mit 1,15 ml (12 mMol) Bortribromid veretzt. Die Mischung wird dann 14 Stunden lang bei Raumtemperatur gerührt, dann mit 30 ml Ethanol versetzt und weitere 30 Minuten lang gerührt. Anschliessend wird im Vakuum bis zur Trockne eingedampft und das so erhaltene Rohprodukt durch Säulenchromatographie gereinigt.
Ausbeute: % der Theorie,
Schmelzpunkt: °C

| | | | | | | | |
|---|---|---|---|---|---|---|---|
| Ber.: | C | | H | | N | | S |
| Gef.: | | | | | | | |

Beispiel 52

2-(4-Amino-2-methoxy-phenyl)-imidazo[1,2-b]pyridazin

0,3 g (1 mMol) 2-(4-Acetamino-2-methoxy-phenyl)-imidazo-[1,2-b]pyridazin werden 1 1/2 Stunden in 20 ml 6n Salzsäure auf dem Dampfbad erhitzt. Nach Abkühlen und Filtration wird mit konz. Ammoniak alkalisch gestellt. Das ausgefallene Produkt wird abgesaugt, mit Wasser gewaschen und in der Trockenpistole bei 100°C über Kaliumhydroxid getrocknet.
Ausbeute: 71 % der Theorie,
Schmelzpunkt: 197-198°C (Zers.)

| | | | | | | |
|---|---|---|---|---|---|---|
| Ber.: | C | 64,99 | H | 5,03 | N | 23,32 |
| Gef.: | | 65,30 | | 5,00 | | 23,56 |

Beispiel 53

2-(4-Aminosulfonyl-2-methoxy-phenyl)-6-chlor-imidazo[1,2-b]-pyridazin

0,6 g (4,5 mMol) 3-Amino-6-chlor-pyridazin und 0,7 g (2,27 mMol) α-Brom-4-aminosulfonyl-2-methoxy-acetophenon werden in 10 ml Dimethylformamid gelöst, 22 Stunden bei

Raumtemperatur und 30 Minuten bei 50°C gerührt. Anschliessend wird das Dimethylformamid am Wasserstrahlvakuum zur Hälfte abgezogen und der Rückstand auf 50 ml Eiswasser gegossen. Das ausgefallene Produkt wird abgesaugt, mit Wasser gewaschen und in der Trockenpistole bei 100°C über Kaliumhydroxid getrocknet.
Ausbeute: 83 % der Theorie (Zers.),
Schmelzpunkt: 310-311°C

| | | | | | | | | | | |
|---|---|---|---|---|---|---|---|---|---|---|
| Ber.: | C | 46,09 | H | 3,27 | N | 16,54 | Cl | 10,47 | S | 9,47 |
| Gef.: | | 45,98 | | 3,31 | | 16,68 | | 10,72 | | 9,56 |

Beispiel 54

2-(4-Acetamino-2-methoxy-phenyl)-imidazo[1,2-b]pyridazin-hydrobromid

2 g (5 mMol) 2-(4-Acetamino-5-brom-2-methoxy-phenyl)-6-chlor-imidazo[1,2-b]pyridazin werden in 100 ml Dimethylformamid suspendiert, mit 0,28 g (5 mMol) gemahlenem Kaliumhydroxid versetzt und 50 Minuten bei Raumptemperatur und in einer Parr-Apparatur mit 0,5 g 20%igem Pd/C bei einem Wasserstoffdruck von 5 bar hydriert. Anschließend werden 25 ml Wasser zugegeben und vom Katalysator abfiltriert. Die Mutterlauge wird einrotiert und nach Verrühren mit 50 ml Wasser abgesaugt, mit Wasser gewaschen und im Vakuumtrockenschrank bei 50°C über Phosphorpentoxid getrocknet.
Ausbeute: 78 % der Theorie,
Schmelzpunkt: 278-285°C (Zers)
Massenspektrum: $M^+$ = 282 m/e.

Beispiel 55

2-(4-Methylsulfonylamino-2-methoxy-phenyl)-imidazo[1,2-b]-pyridazin

Hergestellt aus 2-(4-Amino-2-methoxy-phenyl)-imidazo[1,2-b]-pyridazin und Methansulfochlorid in Pyridin analog Beispiel 2.
Ausbeute: 82 % der Theorie,
Schmelzpunkt: 231°C

| | | | | | | | | |
|---|---|---|---|---|---|---|---|---|
| Ber.: | C | 52,81 | H | 4,43 | N | 17,60 | S | 10,07 |
| Gef.: | | 52,70 | | 4,61 | | 17,43 | | 10,14 |

Beispiel 56

2-(4-Dimethylaminosulfonyl-2-methoxy-phenyl)-6-chlor-imidazo-[1,2-b]pyridazin

Hergestellt aus 3-Amino-6-chlor-pyridazin und α-Brom-4-dimethylaminosulfonyl-2-methoxy-acetophenon analog Beispiel 1.
Ausbeute: 72 % der Theorie,
Schmelzpunkt: 214-215°C

| | | | | | | | | | | |
|---|---|---|---|---|---|---|---|---|---|---|
| Ber.: | C | 49,11 | H | 4,12 | N | 15,27 | Cl | 9,67 | S | 8,74 |
| Gef.: | | 49,35 | | 4,15 | | 15,24 | | 9,89 | | 8,47 |

Beispiel 57

2-(4-Dimethylaminosulfonyl-2-methoxy-phenyl)-imidazo[1,2-b]-pyridazin

Hergestellt aus 3-Amino-pyridazin und α-Brom-4-dimethylaminosulfonyl-2-methoxy-acetophenon analog Beispiel 1.

Ausbeute: 6 % der Theorie,
Schmelzpunkt: 215°C (Zers.)
Massenspektrum: $M^+$ = 332 m/e

Beispiel 58

2-(4-Methylsulfonylamino-2-methoxy-phenyl)-6-chlor-imidazo-[1,2-b]pyridazin

Hergestellt aus 3-Amino-6-chlor-pyridazin und α-Brom-4-methylsulfonylamino-2-methoxy-acetophenon analog Beispiel 1.
Ausbeute: 86 % der Theorie,
Schmelzpunkt: 253-257°C (Zers.)

| | | | | | | | | | | |
|---|---|---|---|---|---|---|---|---|---|---|
| Ber.: | C | 47,66 | H | 3,71 | N | 15,88 | Cl | 10,05 | S | 9,09 |
| Gef.: | | 47,88 | | 3,67 | | 15,97 | | 10,25 | | 9,27 |

Beispiel 59

2-(4-Aminosulfonyl-2-methoxy-phenyl)-imidazo[1,2-b]pyridazin

Hergestellt aus 2-(4-Aminosulfonyl-2-methoxy-phenyl)-6-chlor-imidazo[1,2-b]pyridazin und Wasserstoff in Gegenwart von Pd/C analog Beispiel 54.
Ausbeute: 16 % der Theorie,
Schmelzpunkt: 267-277°C (Zers.)
Massenspektrum: $M^+$ = 304 m/e.

Beispiel 60

6-(2-Methoxy-4-methylsulfonyloxy-phenyl)-imidazo[2,1-f]-[1,2,4]triazin

55 mg (0,227 mMol) 6-(2-Methoxy-4-hydroxy-phenyl)-imidazo-[2,1-f][1,2,4]triazin werden in 12 ml 1n Natronlauge gelöst. Zur gelborangen klaren Lösung gibt man 0,2 ml Methansulfonsäurechlorid zu und rührt 2 1/2 Stunden bei Raumtemperatur. Anschließend bringt man die Reaktionslösung mittels 1n Salzsäure auf pH 6 und extrahiert 3 mal mit je 25 ml Essigester. Die Essigesterphase wird über Natriumsulfat getrocknet und eingeengt. Der erhaltene Rückstand wird in Methylenchlorid gelöst und auf eine Kieselgel-Säule aufgetragen, anfangs mit Methylenchlorid, später mit Methylenchlorid/Ethanol (50:1) eluiert. Die gewünschten Eluate werden eingeengt, anschliessend mit Äther/Petroläther (1:1) verrieben und die erhaltenen Kristalle abgesaugt.
Ausbeute: 15 mg (20,7 % der Theorie),
Schmelzpunkt: 202-204°C
Massenspektrum: $M^+$ = 320 m/e.

Beispiel 61

2-(2-Methoxy-4-methylaminocarbonyl-phenyl)-imidazo[1,2-a]-pyridin

Hergestellt aus 2-Amino-pyridin und α-Brom-2-methoxy-4-methylaminocarbonyl-acetophenon analog Beispiel 1.
Ausbeute: 50 % der Theorie,
Schmelzpunkt: 235-237°C

| | | | | | | |
|---|---|---|---|---|---|---|
| Ber.: | C | 68,31 | H | 5,37 | N | 14,94 |
| Gef.: | | 68,20 | | 5,48 | | 15,13 |

Beispiel 62

2-(2-Methoxy-4-dimethylaminocarbonyl-phenyl)-imidazo-[1,2-a]pyrimidin

Hergestellt aus 2-Amino-pyrimidin und α-Brom-2-methoxy-4-dimethylaminocarbonyl-acetophenon analog Beispiel 1.
Ausbeute: 49,1 % der Theorie,
Schmelzpunkt: 212-214°C

| | | | | | | |
|---|---|---|---|---|---|---|
| Ber.: | C | 64,85 | H | 5,44 | N | 18,91 |
| Gef.: | | 65,06 | | 5,68 | | 18,73 |

Analog den vorstehenden Beispielen werden folgende Verbindungen erhalten:

2-(4-Aminosulfonyl-phenyl)-imidazo[1,2-a]pyridin

2-(4-Methylaminosulfonyl-phenyl)-imidazo[1,2-a]pyridin

2-(4-Aminocarbonyl-phenyl)-imidazo[1,2-a]pyridin

2-(4-Dimethylaminocarbonyl-phenyl)-imidazo[1,2-a]pyridin

2-(4-Methylaminocarbonyl-phenyl)-imidazo[1,2-a]pyridin

2-(4-Methoxy-3-dimethylaminosulfonyl-phenyl)-imidazo[1,2-a]-pyrazin

2-(2,4-Dimethoxy-5-dimethylaminosulfonyl-phenyl)-imidazo-[1,2-a]pyrazin

7-(4-Methoxy-3-dimethylaminosulfonyl-phenyl)-imidazo[2,1-c]-[1,2,4]triazin

7-(2,4-Dimethoxy-5-dimethylaminosulfonyl-phenyl)-imidazo-[2,1-c][1,2,4]triazin

2-(4-Dimethylaminosulfonyl-2-methoxy-phenyl)-imidazo[1,2-c]-pyrimidin

2-(2-Methoxy-4-aminosulfonyl-phenyl)-imidazo[1,2-c]pyrimidin

2-(2-Methoxy-4-methylaminosulfonyl-phenyl)-imidazo[1,2-c]-pyrimidin

2-(4-Dimethylaminosulfonyl-phenyl)-imidazo[1,2-c]pyrimidin

2-(2-Methoxy-4-methylsulfonamido-phenyl)-imidazo[1,2-c]-pyrimidin

2-(4-Methylsulfonyloxy-phenyl)-imidazo[1,2-c]pyrimidin

2-(2-Methoxy-4-methylsulfonyloxy-phenyl)-imidazo[1,2-c]-pyrimidin

2-(2-Methoxy-4-aminosulfonyl-phenyl)-imidazo[1,2-b]pyridazin,

2-(4-Dimethylaminosulfonyl-2-methoxy-phenyl)-imidazo[1,2-b]-pyridazin

6-Amino-2-(2-methoxy-4-methylsulfonamido-phenyl)-imidazo-[1,2-b]pyridazin

6-Acetamido-2-(2-methoxy-4-methylsulfonamido-phenyl)-imidazo[1,2-b]pyridazin

6-Dimethylamino-2-(2-methoxy-4-methylsulfonamido-phenyl)-imidazo[1,2-b]pyridazin

2-(4-Benzyloxy-2-methoxy-phenyl)-6-chlor-imidazo[1,2-b]pyrid-azin

2-(4-Hydroxy-2-methoxy-phenyl)-imidazo[1,2-b]pyridazin

2-(2-Methoxy-4-methylsulfonyloxy-phenyl)-imidazo[1,2-b]pyridazin

2-(4-Methylsulfonamido-phenyl)-6-methylsulfonyloxy-imidazo-[1,2-b]pyridazin

2-(4-Aminosulfonyl-phenyl)-imidazo[1,2-b]pyridazin

2-(4-Dimethylaminosulfonyl-phenyl)-imidazo[1,2-b]pyridazin

2-(4-Acetamido-phenyl)-6-chlor-imidazo[1,2-b]pyridazin

2,4-Bis(methylmercapto)-6-(4-benzyloxy-2-methoxy-phenyl)-imidazo[2,1-f][1,2,4]triazin

6-(4-Hydroxy-2-methoxy-phenyl)-imidazo[2,1-f][1,2,4]triazin

6-(2-Methoxy-4-methylsulfonyloxy-phenyl)-imidazo[2,1-f]-[1,2,4]triazin

2-(2-Methoxy-4-methylsulfonyloxy-phenyl)-imidazo[1,2-d]-[1,2,4]triazin

2-(2-Methoxy-4-methylsulfonylamido-phenyl)-imidazo[1,2-d]-[1,2,4]triazin

2-(4-Methylsulfonyloxy-phenyl)-imidazo[1,2-d][1,2,4]triazin

2-(2-Methoxy-4-aminosulfonyl-phenyl)-imidazo[1,2-d][1,2,4]-triazin

2-(2-Methoxy-4-methylaminosulfonyl-phenyl)-imidazo[1,2-d]-[1,2,4]triazin

2-(2-Methoxy-4-dimethylaminosulfonyl-phenyl)-imidazo-[1,2-d][1,2,4]triazin

Beispiel A

Tabletten zu 100 mg 2-(2-Methoxy-4-methylsulfonyloxy-phenyl)-imidazo-[1,2-a]pyrazin

Zusammensetzung

1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm

Dem Granulat die restlichen Hilfsstoffe zumischen und Endmischung zu Tabletten verpressen.

Tablettengewicht: 175 mg
Stempel: 8 mm

Beispiel B

Dragées zu 50 mg 2-(2-Methoxy-4-methylsulfonyloxy-phenyl)-imidazo-[1,2-a]pyrazin

Zusammensetzung:

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

 

Wirkstoff und Stärke mit wäßriger Lösung der löslichen Stärke gleichmäßig befeuchten.

Feuchtsiebung: 1,0 mm
Trockensiebung: 1,0 mm,
Trocknung: 50°C im Umlufttrockenschrank

Granulat und restliche Hilfsstoffe mischen und zu Kernen verpressen.

Kerngewicht: 80 mg
Stempel: 6 mm
Wölbungsradius: 5 mm

Die fertigen Kerne werden auf übliche Weise mit einem Zuckerüberzug im Dragierkessel versehen.

Drageegewicht: 120 mg

Beispiel C

Suppositorien zu 75 mg 2-(2-Methoxy-4-methylsulfonyloxy-phenyl)-imidazo-[1,2-a]pyrazin

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

Herstellungsverfahren:

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-(2-Methoxy-4-methylsulfonyloxy-phenyl)-imidazo-[1,2-a]pyrazin

1 Ampulle enthält:

| | |
|---|---|
| Wirksubstanz | 50,0 mg |
| Ethoxylierte Hydroxystearinsäure | 250,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 2-(2-Methoxy-4-methylsulfonyloxy-phenyl)-imidazo-[1,2-a]pyrazin

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Imidazoderivate der allgemeinen Formel

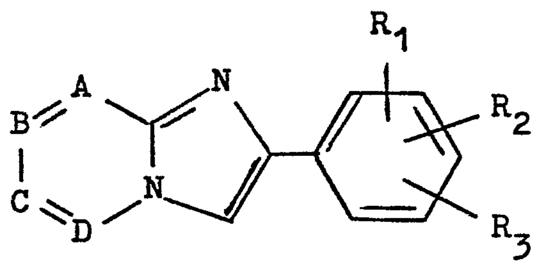

,(I)

in der

keiner, ein oder zwei der Reste A, B, C oder D ein Stickstoffatom,

ein weiterer der Reste A, B, C oder D eine gegebenenfalls durch ein Halogenatom, eine Alkylmercapto-, Alkylsulfonyloxy-, Amino-, Alkylamino-, Dialkylamino- oder Alkanoylaminogruppe substituierte Methingruppe,

ein weiterer der Reste A, B, C oder D eine gegebenenfalls durch eine Alkylmercaptogruppe substituierte Methingruppe und die übrigen der Reste A, B, C oder D Methingruppen,

$R_1$ und $R_2$ zusammen mit zwei dazwischen liegenden Kohlenstoffatomen des Phenylringes einen gegenbenenfalls durch eine Alkoxygruppe substituierten Phenylring und

$R_3$ ein Wasserstoffatom, eine Hydroxy-, Alkoxy- oder Alkylsulfonyloxygruppe oder

der erste der Reste $R_1$, $R_2$ oder $R_3$ eine Phenylalkoxy-, Alkylsulfonyloxy-, Alkylsulfonamido-, N-Alkyl-alkylsulfonamido-, Aminosulfonyl-, Alkylaminosulfonyl- oder Dialkylaminosulfonylgruppe oder in 4-Stellung eine Alkanoylamino-, Aminocarbonyl-, Alkylaminocarbonyl- oder Dialkylaminocarbonylgruppe oder
im Falle der Imidazo[1,2-a]pyrimidine, Imidazo[1,2-b]pyridazine oder Imidazo[2,1-f][1,2,4]triazine oder,
wenn einer oder zwei der Reste A, B, C oder D einen der eingangs erwähnten substituierten Methinreste darstellen, in 4-Stellung auch eine Amino-, Hydroxy-, Methoxy- oder Cyanogruppe,

der zweite der Reste $R_1$, $R_2$ oder $R_3$ ein Wasserstoffatom, eine Hydroxy- oder Alkoxygruppe und der letzte der Reste $R_1$, $R_2$ oder $R_3$ ein Wasserstoffatom oder eine Alkoxygruppe, wobei der Alkylteil bei allen vorstehend erwähnten Resten 1 oder 2 Kohlenstoffatome enthalten kann, und deren Säureadditionssalze.

2. Imidazo[1.2-a]pyrimidine, Imidazo[1.2-c]pyrimidine, Imidazo[1.2-a][1.3.5]triazine, Imidazo[2.1-f][1.2.4]triazine, Imidazo[1.2-a]pyrazine, Imidazo[1.2-d][1.2.4]triazine, Imidazo[1.2-b][1.2.4]triazine, Imidazo[1.2-b]pyridazine, Imidazo[1.2-a]pyridine und Imidazo[2.1-c][1.2.4]triazine der allgemeinen Formel I gemäß Anspruch 1, und deren Säureadditionssalze.

3. Imidazoderivate der allgemeinen Formel I gemäß Anspruch 1, in der

0, 1 oder 2 der Reste A, B, C oder D ein Stickstoffatom,

ein weiterer der Reste A, B, C oder D eine gegebenenfalls durch ein Chlor- oder Bromatom, eine Methylmercapto-, Methylsulfonyloxy-, Amino-, Methylamino-, Dimethylamino- oder Acetamidogruppe substituierte Methingruppe,

ein weiterer der Reste A, B, C oder D eine gegebenenfalls durch eine Methylmercaptogruppe substituierte Methingruppe und

die übrigen der Reste A, B, C oder D Methingruppen,

$R_1$ und $R_2$ zusammen mit zwei dazwischen liegenden Kohlenstoffatomen des Phenylringes einen gegebenfalls durch eine Methoxygruppe substituierten Phenylring und

$R_3$ ein Wasserstoffatom, eine Hydroxy-, Methoxy- oder Methansulfonyloxygruppe oder

der erste der Reste $R_1$, $R_2$ oder $R_3$ eine Acetamido-, Methylsulfonyloxy-, Methylsulfonamido-, N-Methyl-methylsulfonamido-, Aminosulfonyl-, Methylaminosulfonyl-, Dimethylaminosulfonyl-, Aminocarbonyl-, Methylaminocarbonyl- oder Dimethylaminocarbonylgruppe,

der zweite der Reste $R_1$, $R_2$ oder $R_3$ ein Wasserstoffatom, eine Hydroxy- oder Methoxygruppe und

der letzte der Reste $R_1$, $R_2$ oder $R_3$ ein Wasserstoffatom oder eine Methoxygruppe bedeuten, insbesondere jedoch diejenigen Verbindungen, in denen der erste der Reste in 2- oder 4-Position und der zweite der Reste in der verbleibenden 2- oder 4-Position steht, und deren Säureadditionssalze.

4. Imidazoderivate der allgemeinen Formel I gemäß Anspruch 1, in der

0, 1 oder 2 der Reste A, B, C oder D Stickstoffatome und die übrigen der Reste A, B, C oder D Methingruppen,

$R_1$ in 2- oder 4-Stellung eine Methylsulfonyloxy-, Methylsulfonamido-, N-Methyl-methylsulfonamido-, Aminosulfonyl-, Methylaminosulfonyl- oder Dimethylaminosulfonylgruppe,

$R_2$ in der verbleibenden 2- oder 4-Stellung eine Methoxygruppe und

$R_3$ ein Wasserstoffatom darstellen, und deren Säureadditionssalze.

5. 2-(2-Methoxy-4-methylsulfonyloxy-phenyl)-imidazo[1,2-a]-pyrazin und dessen Säureadditionssalze.

6. Physiologisch verträgliche Säureadditionssalze mit anorganischen oder organischen Säuren der Verbindungen gemäß den Ansprüchen 1 bis 5.

7. Arzneimittel, enthaltend eine Verbindung gemäß den Ansprüchen 1 bis 5 oder ein physiologisch verträgliches Säureadditionssalz gemäß Anspruch 6 neben einem oder mehreren inerten Trägerstoffen und/oder Verdünnungsmitteln.

8. Verwendung einer Verbindung gemäß den Ansprüchen 1 bis 5 oder eines physiologisch verträglichen Säureadditionssalzes gemäß Anspruch 6 zur Behandlung von Herzinsuffizienzen.

9. Verfahren zur Herstellung eines Arzneimittels, dadurch gekennzeichnet, daß auf nichtchemischem Wege eine Verbindung gemäß den Ansprüchen 1 bis 5 oder ein physiologisch verträgliches Säureadditionssalz gemäß Anspruch 6 in einen oder mehreren inerten Trägerstoffen und/oder Verdünnungmitteln eingearbeitet wird.

10. Verfahren zur Herstellung der neuen Imidazoderivate der allgemeinen Formel I gemäß den Ansprüchen 1 bis 6, dadurch gekennzeichnet, daß

a) eine Verbindung der allgemeinen Formel

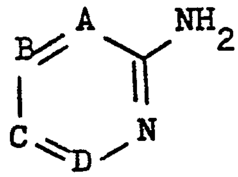
,(II)

in der
A, B, C und D wie in den Ansprüchen 1 bis 5 definiert sind, mit einer Verbindung der allgemeinen Formel

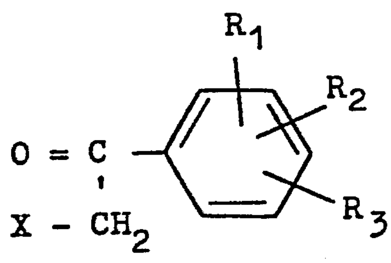
,(III)

in der
$R_1$, $R_2$ und $R_3$ wie in den Ansprüchen 1 bis 5 definiert sind und
X eine nukleophile Austrittsgruppe wie ein Halogenatom, z.B. ein Chlor- oder Bromatom, darstellt, umgesetzt wird oder

b) zur Herstellung von Verbindungen der allgemeinen Formel I, in der einer der Reste $R_1$, $R_2$ oder $R_3$ eine Alkylsulfonyloxy-, Alkylsulfonamido- oder N-Alkyl-alkylsulfonamidogruppe darstellt, eine Verbindung der allgemeinen Formel

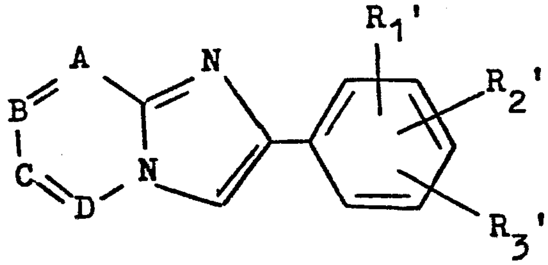
,(IV)

in der A, B, C und D wie in den Ansprüchen 1 bis 5 definiert sind und die Reste $R_1'$, $R_2'$ und $R_3'$ mit Ausnahme der Alkylsulfonyloxy-, Alkylsulfonamido- und N-Alkyl-alkylsulfonamidogruppe die für $R_1$, $R_2$ oder $R_3$ in den Ansprüchen 1 bis 5 erwähnten Bedeutungen besitzen, wobei jedoch einer der Reste $R_1'$, $R_2'$ oder $R_3'$ eine Hydroxy-, Amino-, Methylamino- oder Ethylaminogruppe darstellen muß, mit einer Verbindung der allgemeinen Formel

$$R_4 - SO_2 - Y \qquad ,(V)$$

in der $R_4$ eine Methyl- oder Ethylgruppe und Y eine nukleophile Austrittsgruppe wie ein Halogenatom oder eine Alkoxygruppe, z.B. ein Chlor- oder Bromatom, eine Methoxy-, Ethoxy- oder Benzyloxygruppe, bedeuten, umgesetzt wird oder

c) zur Herstellung von Verbindungen der allgemeinen Formel I, in der mindestens einer der Reste $R_1$, $R_2$ oder $R_3$ eine Alkoxygruppe oder einer der Reste $R_1$, $R_2$ oder $R_3$ eine N-Alkyl-alkylsulfonamidogruppe darstellen, eine Verbindung der allgemeinen Formel

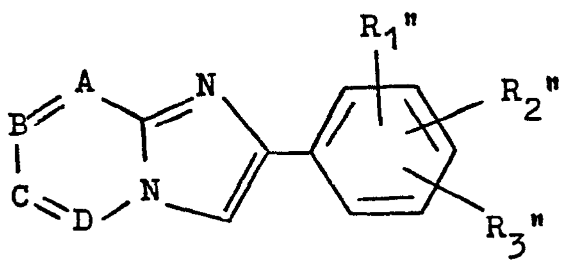

,(VI)

in der
A, B, C und D wie in den Ansprüchen 1 bis 5 definiert sind und
$R_1$", $R_2$" und $R_3$" die für $R_1$, $R_2$ oder $R_3$ in den Ansprüchen 1 bis 5 erwähnten Bedeutungen besitzen, wobei jedoch mindestens einer der Reste $R_1$", $R_2$" oder $R_3$" eine Hydroxy- und/ oder eine Alkylsulfonamidogruppe darstellen muß, mit einer Verbindung der allgemeinen Formel

$$R_4 - Z \qquad ,(VII)$$

in der
$R_4$ eine Methyl- oder Ethylgruppe und

Z eine nukleophile Austrittsgruppe wie ein Halogenatom oder eine Sulfonyloxygruppe, z.B. ein Chlor-, Brom- oder Jodatom, eine Methylsulfonyloxy-, Methoxysulfonyloxy- oder p-Toluolsulfonyloxygruppe, darstellen, alkyliert wird oder

d) zur Herstellung von Verbindungen der allgemeinen Formel I, in der einer der Reste $R_1$, $R_2$ oder $R_3$ eine Aminosulfonyl-, Alkylaminosulfonyl- oder Dialkylaminosulfonylgruppe darstellt, eine Verbindung der allgemeinen Formel

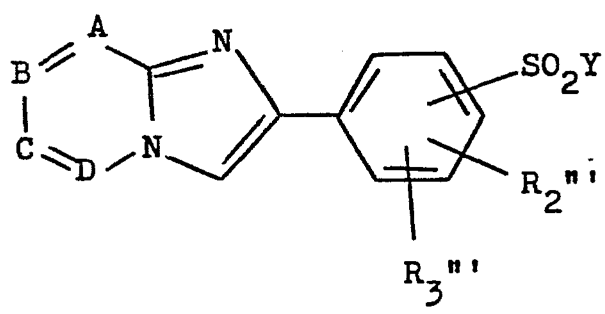

,(VIII)

in der
A, B, C und D wie in den Ansprüchen 1 bis 5 definiert sind, $R_2'''$ und $R_3'''$ die für $R_1$, $R_2$ und $R_3$ mit Ausnahme der Aminosulfonyl-, Alkylaminosulfonyl- oder Dialkylaminosulfonylgruppe in den Ansprüchen 1 bis 5 erwähnten Bedeutungen besitzen und
Y eine nukleophile Austrittsgruppe wie ein Halogenatom oder eine Alkoxygruppe, z.B. ein Chloratom, eine Methoxy- oder Ethoxygruppe, darstellt, mit einem Amin der allgemeinen Formel

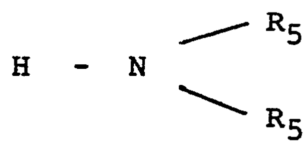

,(IX)

in der
$R_5$ jeweils ein Wasserstoffatom, eine Methyl- oder Ethylgruppe darstellen, umgesetzt wird oder

e) zur Herstellung von Verbindungen der allgemeinen Formel I, in der mindestens einer der Reste $R_1$ bis $R_3$ eine Amino-, Hydroxy- oder Aminocarbonylgruppe darstellt oder enthält, eine Verbindung der allgemeinen Formel

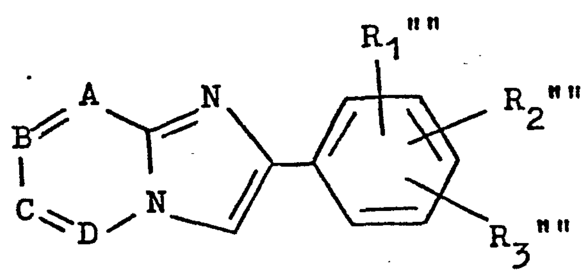

,(X)

in der
A, B, C und D mit Ausnahme der durch ein Halogenatom eine Alkylmercapto-, Alkylsulfonyloxy- oder Phenylalkoxygruppe substituierte Methingruppe die für A, B, C und D die in den Ansprüchen 1 bis 5 erwähnten Bedeutungen aufweisen,

$R_1$"" bis $R_3$"" die für $R_1$ bis $R_3$ die in den Ansprüchen 1 bis 5 erwähnten Bedeutungen aufweisen, wobei jedoch einer der Reste $R_1$"" bis $R_3$"" eine Cyano-, Alkoxy-, Alkanoylamino- oder Alkylsulfonyloxygruppe enthalten oder darstellen muß, hydrolysiert wird oder

f) zur Herstellung von Verbindungen der allgemeinen Formel I, in der mindestens 2 der Reste A, B, C oder D Methingruppen oder mindestens einer der Reste $R_1$ bis $R_3$ ein Wasserstoffatom darstellen muß, ein oder zwei Reste von einer Verbindung der allgemeinen Formel

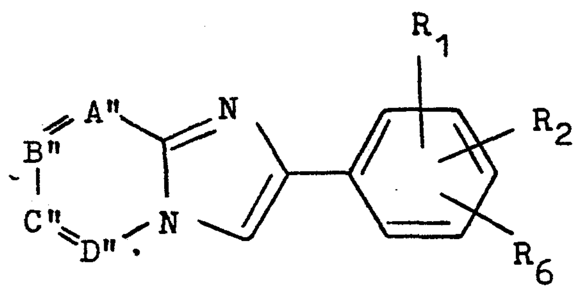

,(XI)

in der

$R_1$ und $R_2$ wie in den Ansprüchen 1 bis 5 definiert sind,

$R_6$ die für $R_3$ in den Ansprüchen 1 bis 5 erwähnten Bedeutungen besitzt und

A", B", C" oder D" die für A, B, C und D die in den Ansprüchen 1 bis 5 erwähnten Bedeutungen aufweisen, wobei jedoch mindestens einer der Reste $R_6$, A", B", C" oder D" einen reduktiv abspaltbaren Rest, z.B. ein Chlor-, Brom- oder Jodatom oder eine Alkylmercaptogruppe, darstellen oder enthalten muß, reduktiv abgespalten wird und

gewünschtenfalls eine so erhaltene Verbindung der allgemeinen Formel I, in der einer der Reste A, B, C oder D eine durch ein Halogenatom substituierte Methingruppe darstellt, mittels Aminolyse in die entsprechende Aminoverbindung der allgemeinen Formel I übergeführt wird oder

eine so erhaltene Verbindung der allgemeinen Formel I in ihr Säureadditionssalz, insbesondere in ihr Säureadditionssalz mit einer physiologisch verträglichen anorganischen oder organischen Säure übergeführt wird.

0185345
Nummer der Anmeldung

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

EP 85116025.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | EP - A1 - 0 113 236 (ELI LILLY AND COMPANY)<br>* Ansprüche 1-3,7-9 *<br>-- | 1,2,6,<br>7,9,10 | C 07 D 487/04<br>C 07 D 471/04<br>A 61 K 31/395<br>//(C 07 D 487/04)<br>(C 07 D 237:00)<br>(C 07 D 235:00)<br>(C 07 D 487/04)<br>(C 07 D 239:00)<br>(C 07 D 235:00)<br>(C 07 D 487/04)<br>(C 07 D 241:00)<br>(C 07 D 235:00)<br>(C 07 D 487/04)<br>(C 07 D 251:00)<br>(C 07 D 235:00)<br>(C 07 D 487/04)<br>(C 07 D 253:00)<br>(C 07 D 235:00)<br>(C 07 D 471/04)<br>(C 07 D 235:00)<br>(C 07 D 221:00) |
| A | DE - A1 - 2 820 938 (MERCK & CO. INC.)<br>* Ansprüche 1,28; Seiten 14,17-19 *<br>-- | 1,2,6,<br>7,9,10 | |
| A | DE - A1 - 2 511 316 (EISAI CO., LTD)<br>* Ansprüche 1,5,6 *<br>---- | 1,2,6,<br>7,9,10 | |
| | | | RECHERCHIERTE SACHGEBIETE (Int Cl.4) |
| | | | C 07 D 487/00<br>C 07 D 471/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9,10
Unvollständig recherchierte Patentansprüche: –
Nicht recherchierte Patentansprüche: 8
Grund für die Beschränkung der Recherche:

Behandlung des menschlichen oder tierischen Körpers durch Therapie, Artikel 52(4) des Europäischen Patentübereinkommens

| Recherchenort | Abschlußdatum der Recherche | Prüfer |
|---|---|---|
| WIEN | 10-03-1986 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument